# EUROPEAN PATENT APPLICATION

(11) **EP 4 653 869 A1**
(43) Date of publication of application: **26.11.2025**
(21) Application number: 24305786.6
(22) Date of filing: 21.05.2024
(51) Int. Cl.: G01N 33/68

(54) **MOLECULES MIMICKING INTERLEUKIN 34 BONE REGULATION ACTIVITY BASED ON THE BINDING OF IL-34 TO BONE MORPHOGENETIC PROTEINS, METHOD OF SCREENING AND PHARMACEUTICAL COMPOSITION**

(71) Applicant: Nantes Université, 44035 Nantes Cedex 1 (FR); Institut de Cancérologie de l'Ouest, 49100 Angers (FR); Centre National de la Recherche Scientifique, 75016 Paris (FR); Institut National de la Santé et de la Recherche Médicale, 75013 Paris (FR); SORBONNE UNIVERSITE, 75006 Paris (FR)
(72) Inventor: HEYMANN, Dominique, 44610 Indre (FR); LEZOT, Frédéric, 44610 Indre (FR); MUÑOZ-GARCIA, Javier, 44850 Ligné (FR)
(74) Representative: Novagraaf Technologies

(57) **Abstract**

The present invention relates to a method of screening for a molecule mimicking interleukin 34 (IL-34) bone regulation activity based on the binding of IL-34 to bone morphogenetic proteins (BMPs), which comprises the steps of:
1) reacting a candidate molecule and BMPs under suitable conditions,
2) detecting the binding of the candidate molecule to BMPs and optionally a bone regulation activity,
3) comparing the binding and optionally the bone regulation activity detected in step 2) to a control, and
4) selecting a molecule capable of binding to BMPs and inducing osteoblast differentiation and activities without activating osteoclast functions.

The present invention also relates to a molecule mimicking IL-34 bone regulation activity based on the binding of IL-34 to BMPs, obtained by the method, and to a pharmaceutical composition comprising the molecule and a pharmaceutically acceptable carrier.

## Description

### Technical field

The present invention refers to a method of screening for a molecule having bone regulation activity based, a molecule obtained by the method, and to a pharmaceutical composition comprising the molecule and a pharmaceutically acceptable carrier.

Therefore, the present invention has utility in medical field.

In the description below, the references into brackets ([ ]) refer to the listing of references situated at the end of the text.

### Background of the Invention

Bone tissue is a subtype of dense connective tissue largely composed of collagen and hydroxyapatite. Populated by three main cell types - osteoblasts, osteoclasts and osteocytes - bone tissue provides protection to other organs, supports the body, and enables movement.

Bone is a dynamic tissue, subject to a constant remodeling process that operates to maintain skeletal strength and health. This remodeling process entails two phases: an osteolysis phase and an osteogenesis phase. In osteolysis, osteoclast precursors are recruited at mineralized surface, are differentiated into active osteoclasts and then erode it by releasing a cocktail of acids and enzymes that degrade collagen and dissolve minerals. This creates small lacunae in the bone. In osteogenesis, osteoblasts deposit new collagenic extracellular matrix used as scaffold for mineral deposition into the lacunae. When osteolysis and osteogenesis are in balance, no net change in bone mass results. Each year, the bone remodeling process replaces approximately 10-20% of a healthy individual's bone throughout the skeleton.

When bone tissue is injured, an ossification process occurs attempting the restoration of tissue's normal function. The healing process generally involves coordinated responses of the bone marrow, bone cortex, periosteum and the surrounding soft tissues, including regulation of cellular proliferation, migration and differentiation. Many signaling molecules, such as fibroblast growth factors (FGF), bone morphogenetic proteins (BMPs), platelet-derived growth factor (PDGF) and vascular endothelial growth factor (VEGF) are involved in the regulation of new bone formation. Through the release of cytokines, hypoxia and vascular disruption, cells are recruited to the fracture site.

BMPs are probably the most important growth factors involved in regenerating bone. They regulate osteogenesis at two different levels: (1) the commitment of skeletal progenitor cells and (2) the maturation of osteoblasts in postnatal development. Specifically, BMP-2 was shown to be effective in inducing osteogenesis. However, treatment of bone defects with BMP proteins (in particular with recombinant BMP-2) is expensive and requires supraphysiological concentrations which run the risk to cause severe side effects like inflammation and the formation of structurally abnormal bone.

Furthermore, in certain disease states, osteolysis is more active than osteogenesis, resulting in a net loss of bone. Such excessive osteolysis may occur in localized areas of the skeleton or more broadly throughout the skeleton. Regardless, bone loss has serious health consequences, including fractures, hypercalcemia, nerve compression syndromes, deformity and pain.

When excessive osteolysis occurs throughout broad areas of the skeleton, it falls under the generic description osteoporosis. Common types of osteoporosis include age-related, post-menopausal, glucocorticoid-induced, diabetes-associated and disuse osteoporosis. Worldwide, osteoporosis presents a staggering problem. Osteoclasts, the cells that mediate excessive osteolysis, operate under the control of numerous cytokines and growth factors. They are multinucleated cells that derive from monocytic precursors. Differentiation of the monocytic precursors into osteoclasts is a complex process that requires both macrophage colony-stimulating factor (M-CSF) and receptor activator of the NF-κB ligand (RANKL). These factors, and all other cellular and humoral requirements for osteoclast differentiation also exist in the microenvironment of bone tumors.

Monocytes/macrophages, which are related to osteoclasts, express M-CSF receptors and are sensitive to M-CSF produced in their microenvironment. M-CSF is then a key cytokine for differentiation of monocyte precursors into osteoclasts.

Inhibiting osteoclast differentiation and function is a desirable approach to treating excessive osteolysis. However, the currently available substances that do so have limited utility, and often cause significant side effects.

For example, calcitonin, a peptide hormone secreted by the thyroid in response to elevated serum calcium, is a well-characterized inhibitor of osteoclast formation and function. However, chronic exposure to calcitonin leads to loss of its inhibitory effects on osteoclasts, through down regulation of the calcitonin receptor expression (mRNA, protein) at the surface of osteoclasts. Additionally, because calcitonin is a protein, it cannot be taken orally, as it would be digested before it could work. While it does not affect other organs or systems in the body, injectable calcitonin may cause an allergic reaction and unpleasant side effects including flushing of the face and hands, urinary frequency, nausea and a skin rash.

Monoclonal antibodies that bind antigens expressed on osteoclast cells also can block osteolysis. However, such antibodies can induce an immune response in patients.

Bisphosphonates also inhibit osteoclast activity, and extensive data exists regarding their use. Despite their benefits, bisphosphonates are poorly absorbed from the gastrointestinal tract and often induce gastrointestinal discomfort. Moreover, bisphosphonates remain in the bone for years, creating a potential of blocking normal bone repair mechanisms for long term exposure.

Immune cell products such as interferon-gamma (IFN-γ), interferon-alpha (IFN-α), oncostatin M, and taxol, suramine and nitric oxide inhibit osteoclast activity as well. However, all of these agents have significant side effects that limit their utility. Interferons can induce flu-like illness, taxol and suramine frequently have severe toxicities associated with gastrointestinal and/or hematopoietic side effects, and nitric oxide can induce vasodilation and low blood pressure.

Thus, a need exists for alternative solutions in regulation of bone remodeling. The present invention fulfills these and other needs.

### Description of the invention

The Applicant has found that bone cell differentiation is regulated by the crosstalk between BMP-2 and IL-34. They demonstrated that the interaction between IL-34 and BMP-2 regulates the balance between osteoblast and osteoclast differentiation. More particularly, the combination of both factors represses osteoclast formation and speeds up osteoblast differentiation.

Indeed, the Applicant found that the addition of BMP-2 to IL-34 in human mesenchymal stem cells reduced significantly the effect of IL-34 on osteoclastogenesis in a dose dependent manner.

In addition, the Applicant demonstrated that IL-34 regulates the intensity of BMP-2 associated signaling and BMP-2 blocks the M-CSFR dependent signaling induced by IL-34.

It was also shown by the Applicant that IL-34 binds to BMP-2 in the wrist epitope.

Accordingly, the present invention provides a method of screening for a molecule mimicking interleukin 34 (IL-34) bone regulation activity based on the binding of IL-34 to bone morphogenetic proteins (BMPs), which comprises the steps of:
1) reacting a candidate molecule and BMPs under suitable conditions,
2) detecting the selective binding of the candidate molecule to BMPs and optionally a bone regulation activity,
3) comparing the binding and optionally the bone regulation activity detected in step 2) to a control, and
4) selecting a molecule capable of binding to BMPs and inducing osteoblast differentiation and activities without activating osteoclast functions.

"BMPs" or "Bone morphogenetic proteins" refers herein to any BMP belonging to this group of growth factors also known as cytokines and as metabologens, that are able to induce the formation of bone and cartilage. It may be at least one BMP protein selected among BMP-1, BMP-2, BMP-3, BMP-4, BMP-5, BMP-6, BMP-7, BMP-8a, BMP-8b, BMP-10, BMP-11, BMP-15, GDF-1, GDF-2, GDF-3, GDF-5, GDF-6, GDF-7, GDF-8, GDF-9, GDF-10 and GDF-11. Preferably, BMPs are at least one chosen among BMP-2, BMP-4 and BMP-7. Even more preferably, it refers to BMP-2.

"Molecules mimicking interleukin 34 (IL-34) bone regulation activity" refers herein to molecules mimicking the binding activities of IL-34 to BMPs without activation of the MCSF-R pathway and having a bone regulation activity. In other words, it refers to molecules capable of binding to BMPs and inducing osteoblast differentiation and activities without activating osteoclast functions. Such molecules may be an agonist molecule for the binding of IL-34 to BMPs, or a molecule regulating the binding of interleukin-34 to BMPs. The bone regulation activity is a partial or total loss of the activation of IL-34-induced bone degradation via the activation of MCSF-R (Macrophage colony-stimulating factor receptor) and/or an increase of the activities of BMPs on bone formation. For example, a partial or total loss of the activation of IL-34-induced bone degradation amounts to at least 10 %, preferably at least 20 %, more preferably at least 30 %, 40 %, 50 %, 60 %, 70 %, 80 % or 90 %, and even 100 % of the control. For example, an increase of the activities of BMPs on bone formation amounts to at least 110 %, preferably at least 125 %, more preferably at least 150 %, 160 %, 170 %, 180 % or 190 %, still more preferably at least 200 % and most preferably at least 300 % of the control, as defined below.

"Candidate molecule" refers herein to any test substance or test compound of any chemical nature. It may already be known as a drug or medicament for a disease. Alternatively, it may be a known chemical compound not yet known to have a therapeutic effect or so far unknown chemical compound. The candidate molecule may be also a mixture of test substances or test compounds. For example, candidate molecule may be a recombinant IL-34, a mutated IL-34, a portion of IL-34, i.e. a IL-34 peptide, or an aptamer. The mutation may be any mutation as long as the mutated IL34 has a bone regulation activity, preferentially an almost equal or better bone regulation activity than wild type (WT) IL34. The mutated IL-34 may be mutated by deletion, substitution and/or insertion of at least one amino acid. For example, the mutation can include the introduction, deletion, and/or substitution of 1, 5, 10, 11, 12, 13, 14, 15, 16, 17, 18, 19, 20, 21, 22, 23, 24, 25, 26, 27, 28, 29, 30, 35, 40, 45, 50, or X amino acid(s). For example, it may be at least one substitution of IL-34 amino acids chosen among F₄₀ by S, E₁₀₃ by K, Q₁₀₆ by K, L₁₀₉ by R, N¹²⁸ by K, N₁₅₀ by K, S₁₈₄ by A, L₁₈₆ by Q, N₁₈₇ by D. The portion of IL34 may be any portion as long as the truncated IL34 has a bone regulation activity, preferentially an almost equal or better bone regulation activity than WT IL34. For example, it may be peptides from amino acids 44-54 and 111-127 of IL-34 sequence.

In the context of the present invention, of the screening method of the present invention, the candidate molecule may be provided in form of a chemical compound library. Chemical compound libraries include a plurality of chemical compounds and have been assembled from any of multiple sources, including chemical synthesized molecules or natural products, or have been generated by combinatorial chemistry techniques. They are especially suitable for high-throughput screening and may be comprised of chemical compounds of a particular structure or compounds of a particular organism such as a plant. In the context of the present invention, the chemical compound library is preferably a library comprising proteins and polypeptides or small organic molecules.

In step 1) of the method of the invention, the candidate molecule may be contacted with BMPs for a time and under reaction conditions suitable for allowing binding to BMPs and/or regulating bone activity, if any. The reacting medium may be in a cellular system or a cell-free system, as appropriate under the prevailing conditions. The reacting medium may also comprise further elements such as means for detecting the effect of a candidate molecule in order to identify an agent as bone activity regulator. Suitable means for detection of the effect of a regulator are detailed throughout the present description. Suitable conditions include appropriate temperature and solution to avoid e.g. denaturation of proteins involved or to maintain viable cells, if present. Suitable conditions will depend on the particular reacting medium chosen and the skilled person will be able to select the same based on his general knowledge.

After step 1) of reacting the candidate molecule and BMPs under suitable conditions, a step 2) of detecting the selective binding of the candidate molecule to BMPs and/or detecting a bone regulation activity.

For these purposes, any detection system usually used for similar binding detection and/or activity may be used according to the current knowledge of the skilled person. Preferably, the method is adapted for high-through put screening. In this method a large number of compounds is screened against the agents in either cell- free or whole-cell assays.

Regarding the detection of the binding of the candidate molecule to BMPs, any method for investigating protein-protein interactions known in the state of the art may be used. It may be for example Western blotting, co-immunoprecipitation, ELISA (enzyme linked immuno sorbent assay), DELFIA (dissociation enhanced lanthanide fluoro immuno assay), SPA (scintillation proximity assay), flashplate assay, ALPHA (amplified luminescent proximity homogenous assay), bimolecular fluorescence complementation, affinity electrophoresis, label transfer, phage display, tandem affinity purification, chemical cross-linking, strep protein interaction experiment, quantitative immunoprecipitation combined with knock-down, proximity ligation assay, surface plasmon resonance, dual polarization interferometry, static light scattering, dynamic light scattering, flow-induced dispersion analysis, fluorescence polarization/anisotropy, fluorescence correlation spectroscopy, fluorescence resonance energy transfer, bio-layer interferometry, multi-nuclear relaxation measurements or 2D-FT NMR spectroscopy, isothermal titration calorimetry, microscale thermophoresis, Rotating cell-based ligand binding assay using radioactivity or fluorescence, and single color reflectometry.

Advantageously, the binding site(s) of the candidate molecule to BMPs are investigated, possibly to be compared to the binding site of IL-34 to BMPs.

It has to be noted that steps 1 and 2) of detecting the binding of the candidate molecule to BMPs may be carried out in totality or in part in vivo, in vitro or in silico.

In an embodiment of the invention, it is also tested whether the candidate molecule can bind to macrophage colony-stimulating factor receptor (MCSF-R). Preferably, the selected molecule is not able to bind to MCSF-R.

Regarding the detection of a bone regulation activity, any method known by the skilled person may be used. It may be done in vitro, for example by an osteoclast differentiation assay, an osteoblastic differentiation assay, measurement of Smad 1/5 signaling as the transcription factors Smad1 and Smad5 are the pivotal intracellular effectors of the BMP family of proteins, mRNA and/or protein expression of factors associated to bone formation (for example: Runx2; Bone Morphogenetic Protein, Bone Sialoprotein, osteocalcin, or osteopontin), bone resorption (for example: NFaTC₁, cathepsin K, or MMP-9), or in vivo in animal models of bone loss, for example osteoporotic mice after ovariectomy, osteolytic bone tumors, analyzed by xRay micro tomodensitometry and histology of bones. It has to be noted that when present, the step of detecting/comparing a bone regulation activity may be realized at any time of the method of the invention, e.g. before or after detection of the binding of the candidate molecule to BMPs, and/or before or after comparing the binding of the candidate molecule to BMPs. This can help to confirm, if needed, that the molecule capable of binding to BMPs also has a bone regulation activity.

Exemplary test systems and their use are described in Example 1.

In the context of the invention, the detection may be qualitative and/or quantitative. Therefore, in step 3), in the case of a qualitative detection, the comparison may be a differential sensing of the binding and/or of the bone regulation activity compared to a control. In the case of a quantitative detection, the comparison is made with a "normal" value. In these purposes, the control may be the statistically normal amount of bone regulation activity of IL-34, notably due to its binding to BMPs, in non-pathological human bone tissue or in a test cell, i.e. the same cell not contacted with the candidate molecule. The person skilled in the art knows statistical procedures to assess whether two values are significantly different from each other such as Student's t-test or chi-square tests.

Advantageously, the binding site(s) of the selected candidate molecule to BMPs may be the same or almost the same as the one of IL-34, in order to mimick, regulate and/or compete the binding of IL-34 to BMPs. Preferably, the candidate molecule binds to BMPs in at least one wrist epitope. For example, the candidate molecule selectively binds to residues F₃₀₆, W₃₁₁, W₃₁₄, Y₃₂₁, M₃₇₁, Y₃₈₅, M₃₈₈ of BMP-2. Alternatively, the binding site may be different, as long as the bone regulation activity is maintained.

Another object of the invention relates to a molecule mimicking IL-34 bone regulation activity based on the binding of IL-34 to BMPs, obtained by a method as defined in anyone of the preceding claims. Advantageously, the molecule is as the technical features described above for the selected candidate molecule. As mentioned above, the molecule may be a recombinant, a mutated IL-34 (for example by deletion, substitution and/or insertion of at least one amino acid) or a IL-34 peptide. For example, it may be regions ⁴⁴KLQYRSRQYM⁵⁴ (SEQ ID NO: 1) and ¹¹¹EGHPSWKYLQEVETLLL¹²⁷ (SEQ ID NO: 2) in particular amino acids K₄₄ L₄₅ Q₄₆ Y₄₇ R₅₀ L₅₁ Q₅₂ Y₅₃ M₅₄ H₁₁₃ P₁₁₄ S₁₁₅ K₁₁₇ L₁₁₉ V₁₂₂ L₁₂₅ L₁₂₆ of IL-34.

Another object of the invention relates to a pharmaceutical composition comprising a molecule as defined above or a pharmaceutically active salt thereof, and a pharmaceutically acceptable carrier.

The pharmaceutical composition of the invention may be used for example in the therapeutic or prophylactic treatment of bone loss, such as osteoporosis, multiple sclerosis, primary bone tumors, bone metastases, multiple myeloma, inflammatory conditions of joint including osteoarthritis, psoriatic arthritis and rheumatoid arthritis, infectious diseases, side effects of radiation therapy, temporal mandibular joint or inflammatory condition resulting from bone and cartilage damage, trauma, orthopedic surgery, infection or other disease process.

The molecule may be formulated in the pharmaceutical composition in accordance with standard pharmaceutical practice for use in a therapeutic or prophylactic treatment in mammals including humans. Therefore, pharmaceutical compositions of the present invention include combinations of at least one molecule as described above, and one or more pharmaceutically acceptable carrier, diluent, or excipient. The phrase "pharmaceutically acceptable" indicates that the substance or composition must be compatible chemically and/or toxicologically, with the other ingredients of the formulation, and the mammal being treated therewith. Suitable carriers, diluents and excipients are well known to those skilled in the art and include materials such as carbohydrates, waxes, water soluble and/or swellable polymers, hydrophilic or hydrophobic materials, gelatin, oils, solvents, water and the like. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, saline, cyclodextrin, ethanol, propylene glycol, polyethylene glycols (e.g., PEG 400, PEG 300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents and flavoring agents.

The pharmaceutical compositions of the invention may be prepared using conventional dissolution and mixing procedures. For example, the bulk drug substance may be dissolved in a suitable solvent in the presence of one or more of the excipients described above.

The pharmaceutical compositions of the invention may be administered by any route appropriate to the condition to be treated, which includes oral and parenteral routes, the latter including subcutaneous, intramuscular, intravenous, intraarterial, intradermal, intrathecal, epidural, and infusion techniques. It will be appreciated that the preferred route may vary with for example the condition of the recipient. Where the composition is administered orally, it may be formulated as tablets, pills, hard or soft e.g., gelatin capsules, cachets, troches, lozenges, aqueous or oil suspensions, dispersible powders or granules, emulsions, syrups or elixirs each containing a predetermined amount of each active agent, with a pharmaceutically acceptable carrier, glidant, or excipient. Where the composition is administered parenterally, it may be formulated with a pharmaceutically acceptable parenteral vehicle or diluent, and in a unit dosage injectable form, as detailed below. For example, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

This invention is further illustrated by the following examples with regard to the annexed drawings that should not be construed as limiting.

### Brief description of the figures

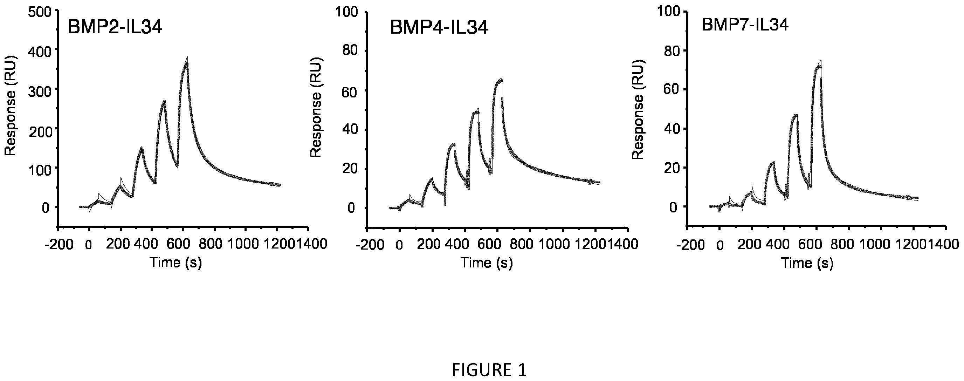

- Figure 1: represents the binding properties of IL-34 to various BMP2, BMP4 and BMP7, in time (s) by response (RU).

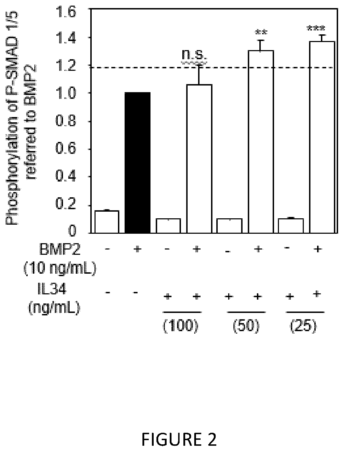

- Figure 2: represents the measurement of SMAD1/5 (phosphorylation of P-SMAD 1/5 referred to BMP2) signaling by Alpha SureFire^{®} Technology, for BMP2 (± 10 ng/mL) and IL34 at 0, 100, 50 and 25 ng/mL.

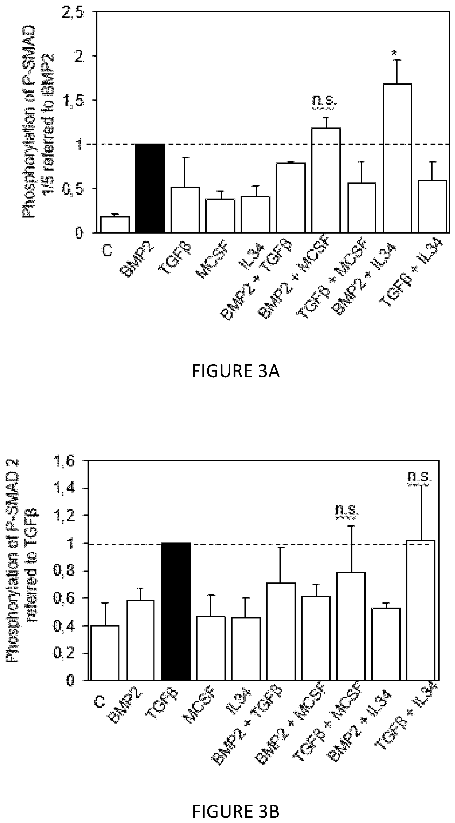

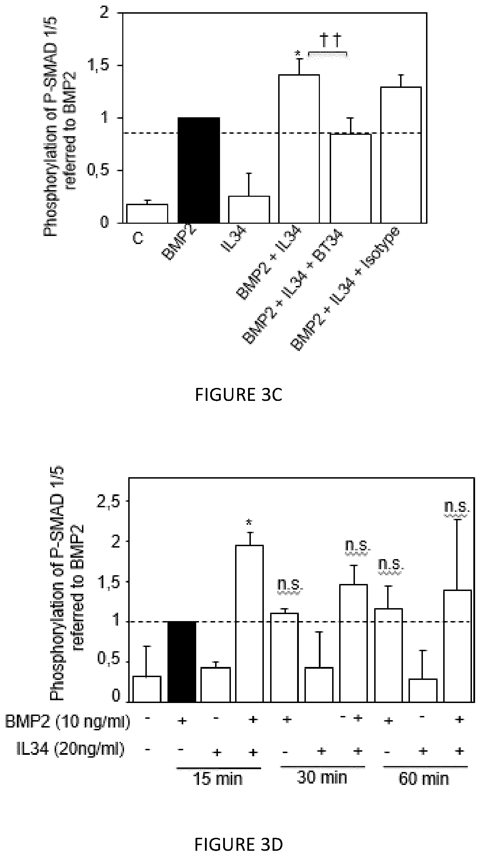

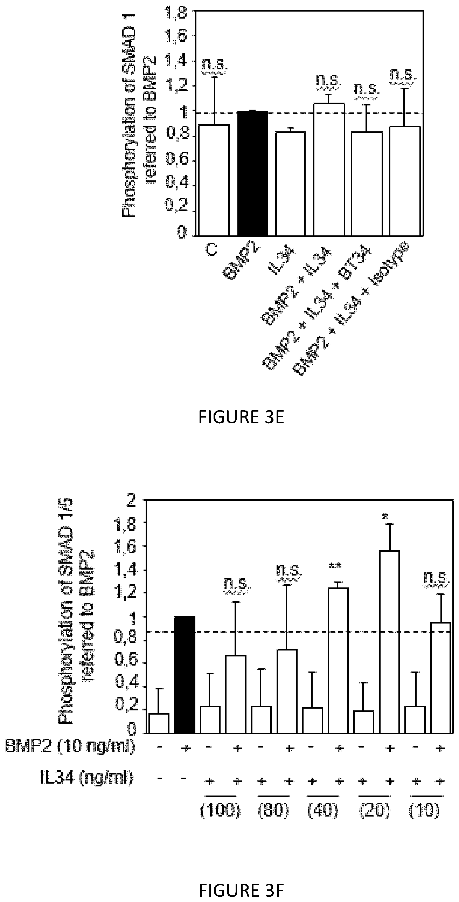

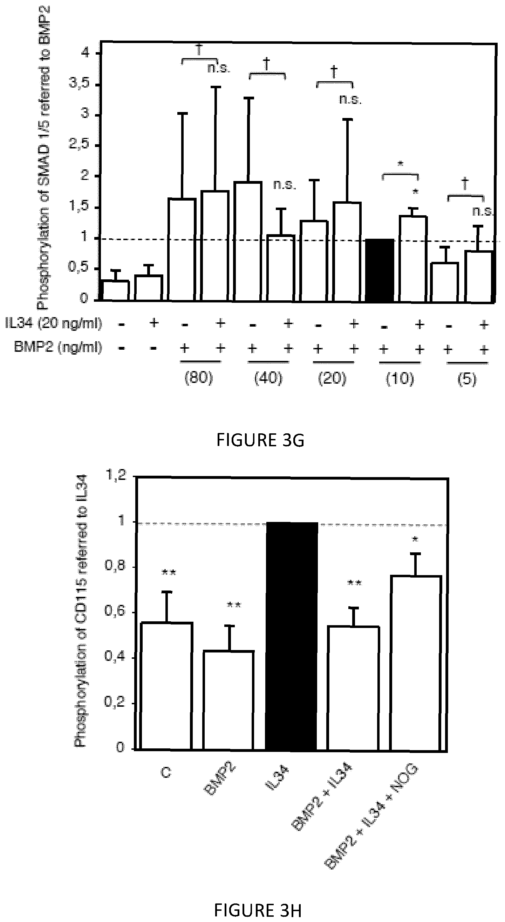

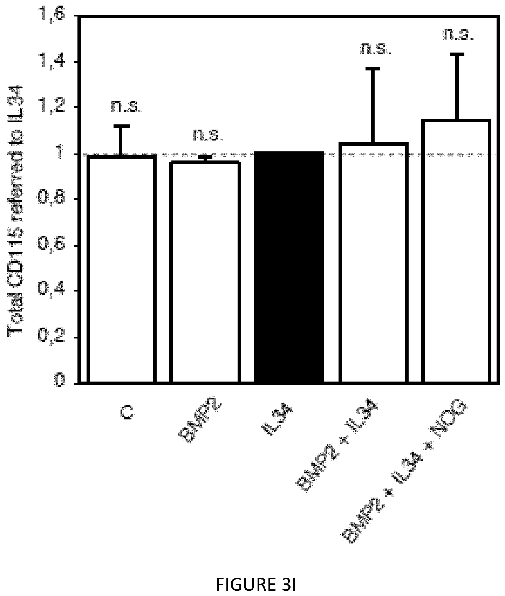

- Figure 3: represents: A) the measurement of phosphorylation of P-SMAD 1/5 referred to BMP2 by Alpha SureFire^{®} Technology, for control, BMP2, TGFβ, MCSF, IL34, BMP2+TGFβ, BMP2+MCSF, TGFβ+MCSF, BMP2+IL34, TGFβ+IL34. B) the measurement of phosphorylation of P-SMAD 2 referred to TGFβ by Alpha SureFire^{®} Technology, for control, BMP2, TGFβ, MCSF, IL34, BMP2+TGFβ, BMP2+MCSF, TGFβ+MCSF, BMP2+IL34, TGFβ+IL34. C) the measurement of phosphorylation of P-SMAD 1/5 referred to BMP2 by Alpha SureFire^{®} Technology, for control, BMP2, IL34, BMP2+IL34, BMP2+IL34+BT34, BMP2+IL34+isotype. D) the measurement of phosphorylation of P-SMAD 1/5 referred to BMP2 by Alpha SureFire^{®} Technology, for BMP2 (± 10 ng/mL) and IL34 (± 20 ng/mL) at 15 min, 30 min and 60 min. E) the measurement of phosphorylation of SMAD 1 referred to BMP2 by Alpha SureFire^{®} Technology, for control, BMP2, IL34, BMP2+IL34, BMP2+IL34+BT34, BMP2+IL34+isotype. F) the measurement of phosphorylation of SMAD 1/5 referred to BMP2 by Alpha SureFire^{®} Technology, for BMP2 (± 10 ng/mL) and IL34 at 0, 100, 80, 40, 20 and 10 ng/mL. G) the measurement of phosphorylation of SMAD 1/5 referred to BMP2 by Alpha SureFire^{®} Technology, for IL34 (± 20 ng/mL) and BMP2 at 0, 80, 40, 20, 10 and 5 ng/mL. H) the measurement of phosphorylation of CD115 referred to IL34 by Alpha SureFire^{®} Technology, for control, BMP2, IL34, BMP2+IL34, BMP2+IL34+NOG. I) the measurement of total CD115 referred to IL34 by Alpha SureFire^{®} Technology, for control, BMP2, IL34, BMP2+IL34, BMP2+IL34+NOG.

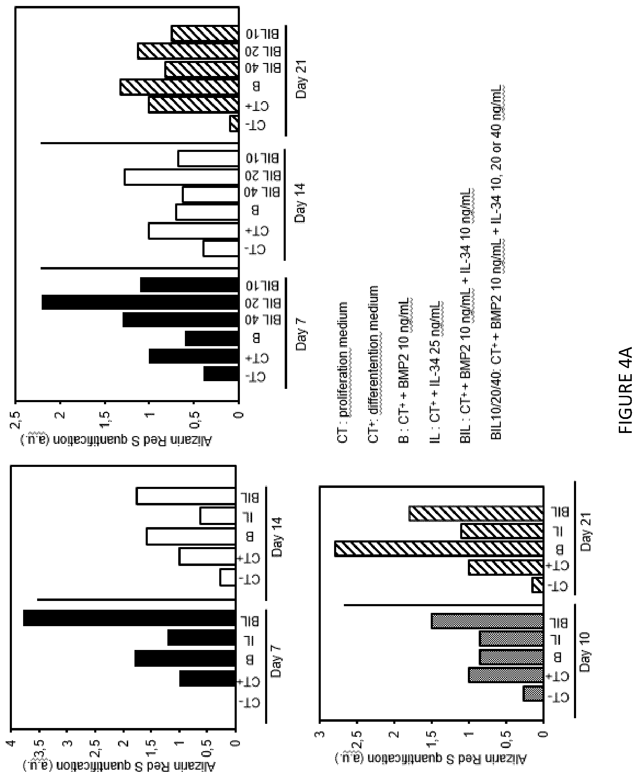

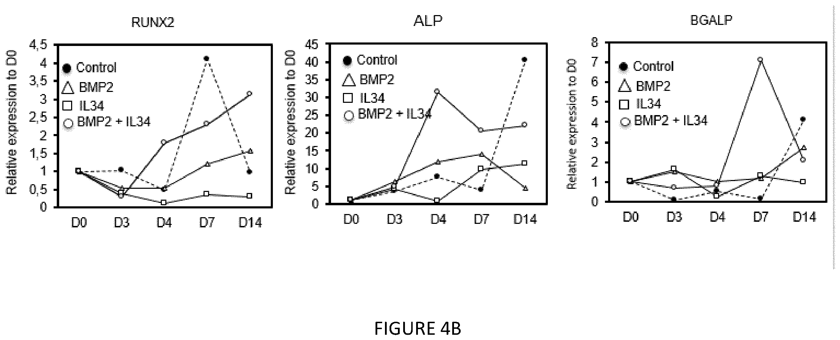

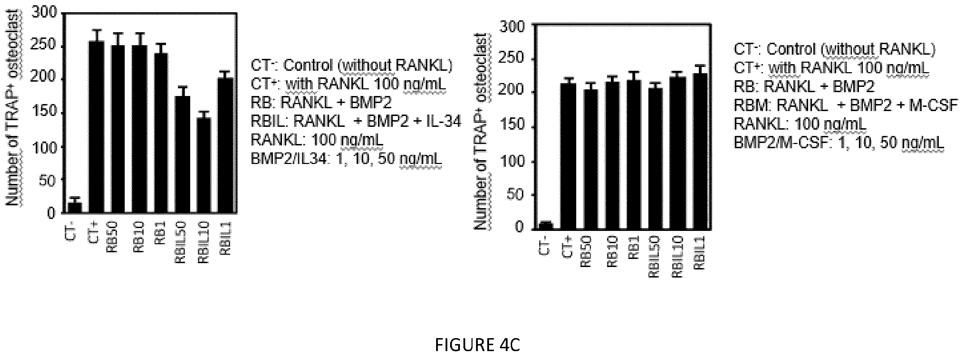

- Figure 4: represents: A) Human mesenchymal stem cells were differentiated in the presence of absence of BMP-2 and IL-34. Three independent cell batches were analyzed for differentiation of mesenchymal stem cells into osteoblast by alizarin red staining (Alizarin red S quantification in a.u.), for CT- (proliferation medium), CT+ (differentiation medium), B (CT+ + BMP2 10ng/mL), IL (CT+ + IL34 25 ng/mL), BIL (CT+ + BMP2 10 ng/mL + IL34 10 ng/mL), BIL 10/20/40 (CT+ + BMP2 10 ng/mL + IL34 10, 20 or 40 ng/mL). B) Analysis of the expression of the genes associated with osteoblast differentiation (RUNX2, ALP, BGALP) by RTqPCR (relative gene expression compared to D0), at D0, D3, D4, D7 and D14, for control (full circle, dotted line), BMP2 (empty triangle), IL34 (empty square) and BMP2+IL34 (empty circle). C) Assessment of the effect of both factors on osteoclast differentiation by following the differentiation of purified human CD14⁺ cells into TRAP⁺ multinucleated cells (number of TRAP+ osteoclast) for : left panel: CT- (control without RANKL), CT+ (with RANKL 100 ng/mL), RB (RANKL + BMP2 50, 10 and 1 ng/ml), RBIL (RANKL + BMP2 50, 10 and 1 ng/ml + IL34 50, 10 and 1 ng/ml), and for : right panel: CT- (control without RANKL), CT+ (with RANKL 100 ng/mL), RB (RANKL + BMP2 50, 10 and 1 ng/mL) and RBM (RANKL + BMP2 50, 10 and 1 ng/mL + MCFS 50, 10 and 1 ng/mL).

### Examples

### Example 1: The binding of IL-34 to members of the BMP-2 cytokine family abolishes the activation of IL-34-induced bone degradation via the activation of MCSF-R and stimulates the activities of BMP-2 cytokines on bone formation

### Material and methods

### Reagents

Recombinant human Macrophage-Colony Stimulating Factor (M-CSF), human interleukin-34 (IL-34), human M-CSF receptor (M-CSFR/CD115), human TGF-β1, human bone morphogenetic protein 2 (BMP-2), human bone morphogenetic protein 4 (BMP-4), human bone morphogenetic protein 7 (BMP-7), human Noggin, human TRANCE (RANKL) and antibody anti-human M-CSFR, Anti-Phosho-M-CSFR (Y723) were obtained from R&D Systems (Abingdon, UK). Anti-human IL34 (BT-34) mouse IgG1 monoclonal antibody was purchased from Diaclone (Besançon, France). Antibodies directed against human Smad1 (D59D7), human Smad2 (D43B4), anti Phospho-Smad1/5(ser463/465) (41D10), anti-phospho-Smad2 (Ser465/467) (138D), β-Actin (8H10D10), and HRP-conjugated secondary antibodies were purchased from Cell Signalling (Ozyme, Saint Quentin Yvelines, France). Antibodies against p-CD115 (Y723) (ref#AF3268) and CD115 (6710) were purchased from R&D systems. AlphaLISA^{®} SureFire^{®} Ultra Total SMAD1 and p-SMAD1 (Ser463/465) Assay kits were purchased from PerkinElmer (Villebon-sur-Yvette, France).

### Cell cultures

The cell lines used in the present study were purchased from the American Tissue Cell Collection (ATCC, Molsheim, France). HEK293 (HEK) transfected with the pCDNA3 empty plasmid or the pCDNA3 plasmid containing the M-CSFR gene as described by Segaliny et al., 2015 (Ségaliny et al, Syndecan-1 regulates the biological activities of interleukin-34. Biochim Biophys Acta 2015;1853(5):1010-21. doi: 10.1016/j.bbamcr.2015.01.023 ([1])). Osteosarcoma MNNG/HOS (ATCC@ Number: CRL-1547^{™}) were cultured in Dulbecco's Modified Eagle's Medium (DMEM, Lonza, Levallois-Perret, France) supplemented with 10% foetal bovine serum (FBS; Hyclone Perbio, Bezons, France) and 2 mmol/L of L-glutamine.

### Human osteoclast differentiation

CD14⁺ cells were initially isolated from human peripheral blood donors provided by the French blood bank institute (Etablissement Français du Sang, Nantes, France, authorization number: NTS 2000-24), by using MACS microbeads (MiltenyiBiotec, Bergisch Gladbach, Germany) as previously described (Baud'huin et al, Interleukin-34 is expressed by giant cell tumours of bone and plays a key role in RANKL-induced osteoclastogenesis. J Pathol. 2010;221(1):77-86. doi: 10.1002/path.2684 ([2])). For osteoclast differentiation, CD14⁺ cells were cultured in alpha-MEM (Lonza) supplemented with 10% human serum (Invitrogen, France) and in the presence of human M-CSF (25 ng/mL) or human IL34 (100 ng/mL) +/- human BMP2 (40 or 100 ng/mL) for 3 days. Then cells were treated with same molecules in the presence of human RANKL (100 ng/mL) for 11 days. Medium was renewed every 3 days. After 11 days of treatment, osteoclasts were analyzed by Acid Phosphatase (TRAP) staining kits (Sigma Aldrich, Saint-Quentin Fallavier, France). TRAP⁺ multinucleated cells with 3 nuclei and more were considered as osteoclasts and were manually enumerated.

### Human osteoblastic differentiation

Human mesenchymal stem cells (hMSC) were purchased from Lonza (France). Osteoblast differentiation assays were performed as previously described (Gobin et al. BYL719, a new α-specific PI3K inhibitor: single administration and in combination with conventional chemotherapy for the treatment of osteosarcoma. Int J Cancer. 2015;136(4):784-96. doi: 10.1002/ijc.29040 ([3])). Briefly, hMSC were cultured in DMEM was supplemented 10% of FBS, vitamin D3 (10⁻⁸ M; Sigma) and dexamethasone (10⁻⁷ M; Sigma). After 3 days, ascorbic acid (50 ng/mL; Sigma) and β-glycerophosphate (10 mM; Sigma) were added to allow mineralization detected by alizarin red-S staining for three weeks. Images were captured using a stereomicroscope (Stemi 2000-C; Zeiss), and mineralized surfaces were quantified using Qwin software (Leica, Germany). Mineralization process was carried out in the presence or absence of human cytokine IL-34 (25 ng/mL), BMP2 (10 ng/mL) or combination of both molecules for 3 weeks. RNA samples were collected at days 3, 4, 14 and 21 after the induction of differentiation.

### Western blot

The cells were collected in a RIPA buffer (10 mM Tris pH 8, 1 mM EDTA, 150 mMNaCl, 1% NP40, 0.1% SDS containing a cocktail of protease and phosphatase inhibitors Halt^{™} (Thermo Fisher, Waltham, MA, USA). The protein concentration was determined using a BCA (bicinchoninic acid) method by BC Assay Protein Quantitation Kit (Interchim, Montluçon, France). 50 µg of protein extracts were prepared in a Laëmmli buffer (62.5 mM Tris-HCl, pH 6.8, 2% SDS, 10% glycerol, 5% 2-mercaptoethanol, 0.001% bromophenol blue) and then separated by SDS-polyacrylamide gel electrophoresis. After electrophoretic transfer, the immobilon-P membranes (Millipore, Molsheim, France) were blotted with the antibodies referenced in the "Reagents" section. The membranes were then probed with secondary antibodies coupled with horseradish peroxidase. Antibody binding was visualised with an enhanced chemiluminescence (ECL) kit Clarity^{™} Western ECL Substrate (Bio-Rad, Marnes-la-Coquette, France). The luminescence was detected with a ChemiDoc MP Imaging System (Bio-Rad). Blots images and semiquantitative analysis were done using ImageJ software (USA).

### Smad 1/5 signaling measured by Alpha SureFire^{®} Technology

Direct quantification analysis of cell signaling was performed by using Alpha SureFire^{®} Technology from PerkinElmer in a Victor^{®} Nivo^{™} multimode microplate reader (PerkinElmer, Villebon-sur-Yvette, France).

### Biacore assays

All recombinant proteins used in Biacore assays were purchased from R&D System (UK). All SPR experiments were performed on a T200 apparatus (Cytiva) at 25 °C in PBS pH7.4 containing 0.05% surfactant P20. Human recombinant BMP2, BMP4 and BMP7 proteins were immobilized (1500- 2300 RU) at pH 4.5 on CM5-S sensor chip by amine coupling following the manufacturer's instructions (Cytiva, Velizy-Villacoublay, France). IL34 kinetics were measured using one cycle titration, for five increasing concentrations of recombinant human IL-34 (12.5, 25, 50, 100, 200 nM) were injected during 60s at 100 µL/min on coated BMPs. The last injection was followed by a 600s dissociation time in running buffer. The KD values were evaluated using a bivalent fitting model (T200 Evaluation software 3.2.1, Cytiva). All sensorgrams were corrected by subtracting the low signal from the control reference surface (without any immobilized protein) and blank buffer injections before fitting.

### RNA isolation and real-time PCR

Total RNA was extracted using NucleoSpin^{®} RNA Plus (Macherey-Nagel, Duren, Germany). 1µg of total RNA was used for first strand cDNA synthesis using the OneScript^{®} RT Mix (Ozyme). Real-time PCR was performed on 20 ng of reverse transcribed total RNA (cDNA), 300 nM of primers (QuantiTect Primer^{®} Assays, Qiagen) and PowerUp^{™} SYBR^{™} Master Mix from Applied Biosystems^{™} (Thermo Fisher) in a CFX96 Touch Deep Well Real-Time PCR Detection system from Bio-Rad. Thermal cycle conditions were perform by following manufacture protocol. The analysis was performed with CFX Manager Software (Bio-Rad) using human glyceraldehyde 3-phosphate dehydrogenase (GAPDH), Hypoxanthine Phosphoribosyl transferase 1 (HPRT1) and TATA box binding protein (TBP) as invariant controls (QuantiTect Primer^{®} Assays, Qiagen). Oligonucleotides were designed with Primer-Blast software (NCBI) and purchased from Eurogentec (Eurogentec, Angers, France). The 2-ΔΔCt (cycle threshold) method was used to calculate expression levels. List of primers and gene name symbols with corresponding full names are indicated in Table 1 below.

**Table 1: Qiagen q-PCR human primers**

| **Official full name; Alias** | **Official symbol** | **Gene Globe Id** |
|---|---|---|
| Alkaline phosphatase, liver/bone/kidney | ALP | QT00012957 |
| bone gamma-carboxyglutamate protein | BGLAP | QT00232771 |
| Runt-related transcription factor 2 | RUNX2 | QT00020517 |

### Protein-Protein docking and analysis

Structures of M-CSFR, BMPR1 and IL-34 were extracted from their bound crystallographic forms (Keller et al. Molecular recognition of BMP-2 and BMP receptor IA. Nat Struct Mol Biol. 2004;11(5):481-8. doi: 10.1038/nsmb756 ([4])), 4WRL for M-CSF:M-CSFR1 (Felix et al. Structure and assembly mechanism of the signaling complex mediated by human CSF-1. Structure 2015;23(9):1621-1631. doi: 10.1016/j.str.2015.06.019 ([5])) and 4DKD for IL-34:M-CSFR1 (Ma et al. Structural basis for the dual recognition of helical cytokines IL-34 and CSF-1 by CSF-1R. Structure 2012;20(4):676-87. doi: 10.1016/j.str.2012.02.010 ([6])) Docking experiments were performed using either BMP-2 fixed and the partner protein mobile, or the reverse. ClusPro analysis (Kozakov et al. The ClusPro web server for protein-protein docking. Nat Protoc. 2017;12(2):255-278. doi: 10.1038/nprot.2016.169 ([7])) was performed in balanced mode, only the first 10 binding modes clusters were considered for analysis, the best modes were selected by visual inspection. Interface analyses were performed using the PISA web server (http://www.ebi.ac.uk/pdbe/prot_int/pistart.html) (Krissinel et Henrick, Inference of macromolecular assemblies from crystalline state. Journal of Molecular Biology 2007; 372: 774-797 ([8])). Visualization and superimposition of docking poses and crystallographic structures were done using PyMOL (The PyMOL Molecular Graphics System, Version 2.5 Schrödinger, LLC; Schrödinger, LLC 2015).

### Results

### IL-34 binds to BMP-2 in the wrist epitope

The binding properties of IL-34 to various members of the BMP cytokine family was first assessed by surface plasmon resonance (Biacore T200) (Figure 1A). As shown in Figure 1A, IL-34 bound to immobilized BMP-2 in a dose dependent manner. Similarly IL-34 was able to bind to BMP4 and BMP7 cytokines. The IL-34 binding affinity was around 3.6 x 10⁷M for BMP2 (highest Ka and lowest Kd), significantly higher than for BMP4 and BMP7 with 4.2 x 1 0⁷M and 9.2 x 10⁷M, as shown on the table below:

**Table 2: hIL34 affinity to BMPs**

| | Ka (1/Ms) | Kd (1/s) | KD (M) |
|---|---|---|---|
| BMP2 | 6.31E+04 | 0.0229 | 3.63E-07 |
| BMP4 | 1.10E+05 | 0.0467 | 4.26E-07 |
| BMP7 | 6.08E+04 | 0.0561 | 9.22E-07 |

We assessed the binding capacity of BMP-2 structure orthogonal to IL-34 orientation. The solvent-excluded interface is 720 angstroem², amino acids contributing the most to the interface energy are F₃₀₆, W₃₁₁, W₃₁₄, Y₃₂₁, M₃₇₁, Y₃₈₅, M₃₈₈ for BMP-2 and regions ⁴⁴KLQYRSRQYM⁵⁴-¹¹¹EGHPSWKYLQEVETLLL¹²⁷ (in particular amino acids K₄₄ L₄₅ Q₄₆ Y₄₇ R₅₀ L₅₁ Q₅₂ Y₅₃ M₅₄ H₁₁₃ P₁₁₄ S₁₁₅ K₁₁₇ L₁₁₉ V₁₂₂ L₁₂₅ L₁₂₆) for IL-34. Additionally, hydrogen bonds and salt bridges are found between BMP-2 and IL-34 respectively in residues K383-D190, D312-K55, E376-R73. Interestingly, IL-34 binding to BMP-2 was incompatible with IL-34 binding to CSF-R. Indeed, after superimposition of our IL-34 model bound to IL-34 onto the IL-34/CSF-R crystallographic complex, we can conclude that binding to IL-34 is competitive between BMP-2 and CSF-R.

In addition, complex binding stoechiometry was possible. After superimposition of our docking poses with experimental structures of BMP-2 complexes, where wrist and knuckle sites can be occupied, we observe that IL-34 can occupy one wrist site, and the other one can be occupied by another protein, for instance BMPR1A. Both BMPR1A and IL-34 would compete for BMP-2 binding since their BMP-2 recognition mode is similar.

### IL-34 regulates the intensity of BMP-2 associated signaling and BMP-2 blocks the M-CSFR dependent signaling induced by IL-34

The potential effect of IL-34 on the BMP signaling was firstly assessed by using the human MNNG/HOS osteoblastic-like cell lines by Western blot. We first compared the effect of IL-34 on the cell signaling pathways induced by BMP-2 and TGFβ1 (Figure 3, p<0.01). As expected, BMP-2 and TGFβ1 induced the phosphorylations of SMAD1/5 and SMAD 2 respectively compared to the control without any growth factor. IL-34 and M-CSF did not induce any phosphorylation of both signaling pathways. The addition of IL-34 to BMP-2 was associated to a higher level of pSMAD1 phosphorylation compared to BMP-2 alone and this additive effect was not observed in the presence of TGFβ1 or of M-CSF. The addition of BT34 anti-IL34 blocking antibody abolished the positive effect of IL-34 on BMP-2 induced SMAD1/5 phosphorylation (Figure 3, p<0.01). The synergistic effect of IL-34 on BMP-2 signaling was time dependent with a highest signaling at 15 minutes (Figure 3, p<0.01) and dose dependent with a highest effect at the ratio 20 ng/mL (IL-34) 110 ng/mL (BMP2) (Figure 3D-E, Figure 3). To confirm the effect of IL-34 on BMP-2 signaling in a quantitative manner, SMAD1/5 signalling was measured by Alpha SureFire^{®} Technology. Figure 2 and Figure 3 confirmed the dose dependent impact of IL-34 on the phosphorylation of SMAD1/5 induced by BMP-2. IL-34 is known to bind to CD115 (M-CSFR) and induced its phosphorylation (Baud'huin et al, Interleukin-34 is expressed by giant cell tumours of bone and plays a key role in RANKL-induced osteoclastogenesis. J Pathol. 2010;221(1):77-86. doi: 10.1002/path.2684 ([2])). CD115-overexpressing HEK293 cells were used to assess the functional impact of BMP-2 on IL-34 induced CD115 activation (Figure 3). IL-34 induced the phosphorylation of CD115 in contrast to BMP-2 alone, and the addition of BMP-2 to IL-34 abolished the signal observed in the presence of IL-34 alone. Overall, these data demonstrated the upregulation of the BMP signaling by exogenous IL-34 and the repression of IL-34-dependent CD115 activation by BMP-2 addition.

### The interaction between IL-34 and BMP-2 regulates the balance between osteoblast and osteoclast differentiation

Whether the literature clearly demonstrated that BMP signaling regulates osteoblast differentiation and the regulation of osteoclastogenesis and bone resorption by IL-34, the functional crosstalk between both soluble has never been studied. Their functional crosstalk was then analyzed in bone cell differentiation assays (Figure 4). Human mesenchymal stem cells were differentiated in the presence of absence of BMP-2 and IL-34 (Figure 4A). Three independent cell batches were analyzed and confirmed that BMP-2 induced the differentiation of mesenchymal stem cells into osteoblast as shown by an increased mineralization extracellular matrix revealed by alizarin red staining. On the contrary, IL-34 alone had no effect on osteoblast differentiation. Interestingly, the combination of BMP-2 and IL-34 increased the proosteoblastic effects of BMP-2. This effect was dose and time dependent and was optimal in the presence of 20 ng/mL (IL-34) / 10 ng/mL (BMP2). The expression of the genes associated with osteoblast differentiation was analyzed by RTqPCR (Figure 4B). The data revealed the strong impact of BMP-2 and IL-34 combination on osteoblast differentiation genes. Indeed, *Runx2, ALP, BGALP* expressions were earlier in the presence of both cytokines compared to each factor alone. The addition of IL-34 to BMP-2 accelerated the commitment of mesenchymal stem cells toward osteoblasts. Similarly, the effect of both factors on osteoclast differentiation was assessed by following the differentiation of purified human CD14⁺ cells into TRAP⁺ multinucleated cells (Figure 4C). IL-34 induced the differentiation of osteoclasts in the presence of RANKL similarly to M-CSF and in contrast to BMP-2. Interestingly, the addition of BMP-2 to IL-34 reduced significantly the effect of IL-34 on osteoclastogenesis in a dose dependent manner. Such effect was not observed in the presence of M-CSF. Overall, these data demonstrated the regulation of bone cell differentiation is regulated by the crosstalk between BMP-2 and IL-34. The combination of both factors represses osteoclast formation and speeds up osteoblast differentiation.

### Reference List

1. Ségaliny et al, Syndecan-1 regulates the biological activities of interleukin-34. Biochim Biophys Acta 2015;1853(5):1010-21. doi: 10.1016/j.bbamcr.2015.01.023.
2. Baud'huin et al, Interleukin-34 is expressed by giant cell tumours of bone and plays a key role in RANKL-induced osteoclastogenesis. J Pathol. 2010;221(1):77-86. doi: 10.1002/path.2684.
3. Gobin et al. BYL719, a new α-specific PI3K inhibitor: single administration and in combination with conventional chemotherapy for the treatment of osteosarcoma. Int J Cancer. 2015;136(4):784-96. doi: 10.1002/ijc.29040.
4. Keller et al. Molecular recognition of BMP-2 and BMP receptor IA. Nat Struct Mol Biol. 2004;11(5):481-8. doi: 10.1038/nsmb756.
5. Felix et al.: Structure and assembly mechanism of the signaling complex mediated by human CSF-1. Structure 2015;23(9):1621-1631. doi: 10.1016/j.str.2015.06.019.
6. Ma et al. Structural basis for the dual recognition of helical cytokines IL-34 and CSF-1 by CSF-1R. Structure 2012;20(4):676-87. doi: 10.1016/j.str.2012.02.010.
7. Kozakov et al. The ClusPro web server for protein-protein docking. Nat Protoc. 2017;12(2):255-278. doi: 10.1038/nprot.2016.169.
8. Krissinel et Henrick, Inference of macromolecular assemblies from crystalline state. Journal of Molecular Biology 2007 ; 372: 774-797.

## Claims

1. A method of screening for a molecule mimicking interleukin 34 (IL-34) bone regulation activity based on the binding of IL-34 to bone morphogenetic proteins (BMPs), which comprises the steps of:
1) reacting a candidate molecule and BMPs under suitable conditions,
2) detecting the selective binding of the candidate molecule to BMPs and optionally a bone regulation activity,
3) comparing the binding and optionally the bone regulation activity detected in step 2) to a control, and
4) selecting a molecule capable of binding to BMPs and inducing osteoblast differentiation and activities without activating osteoclast functions.

2. The method according to claim 1, wherein said BMPs are at least one chosen among BMP2, BMP4 and BMP7.

3. The method according to claim 1 or 2, wherein the binding is measured by a method selected among Western blotting, co-immunoprecipitation, bimolecular fluorescence complementation, affinity electrophoresis, label transfer, phage display, tandem affinity purification, chemical cross-linking, strep protein interaction experiment, quantitative immunoprecipitation combined with knock-down, proximity ligation assay, surface plasmon resonance, dual polarization interferometry, static light scattering, dynamic light scattering, flow-induced dispersion analysis, fluorescence polarization/anisotropy, fluorescence correlation spectroscopy, fluorescence resonance energy transfer, bio-layer interferometry, multi-nuclear relaxation measurements or 2D-FT NMR spectroscopy, isothermal titration calorimetry, microscale thermophoresis, Rotating cell-based ligand binding assay using radioactivity or fluorescence, and single colour reflectometry.

4. The method according to any one of the preceding claims, wherein said bone regulation activity is a partial or total loss of the activation of IL-34-induced bone degradation via activation of MCSF-R (Macrophage colony-stimulating factor receptor) and/or an increase of the activities of BMPs on bone formation.

5. The method according to any one of the preceding claims, wherein said molecule is not able to bind to macrophage colony-stimulating factor receptor (MCSF-R).

6. The method according to any one of the preceding claims, wherein said molecule increases BMPs activity.

7. The method according to any one of the preceding claims, wherein said molecule selectively binds to F₃₀₆, W₃₁₁, W₃₁₄, Y₃₂₁, M₃₇₁, Y₃₈₅, M₃₈₈ for BMP-2 and regions ⁴⁴KLQYRSRQYM⁵⁴ (SEQ ID NO: 1)-¹¹¹EGHPSWKYLQEVETLLL¹²⁷ (SEQ ID NO: 2) (in particular amino acids K₄₄ L₄₅ Q₄₆ Y₄₇ R₅₀ L₅₁ Q₅₂ Y₅₃ M₅₄ H₁₁₃ P₁₁₄ S₁₁₅ K₁₁₇ L₁₁₉ V₁₂₂ L₁₂₅ L₁₂₆) for IL-34.

8. The method according to any one of the preceding claims, wherein said candidate molecule is a recombinant IL-34, a mutated IL-34, a IL-34 peptide, or an aptamer.

9. The method according to claim 8, wherein said mutated IL-34 is mutated by deletion, substitution and/or insertion of at least one amino acid.

10. A molecule mimicking IL-34 bone regulation activity based on the binding of IL-34 to BMPs, obtained by a method as defined in anyone of the preceding claims.

11. A molecule according to claim 10, that selectively binds to sequence as defined in claim 7.

12. A molecule according claim 10 or 11, wherein said molecule is recombinant IL-34, a mutated IL-34, a IL-34 peptide, or an aptamer.

13. A molecule according to claim 12, wherein said mutated IL-34 is mutated by deletion, substitution and/or insertion of at least one amino acid.

14. A pharmaceutical composition comprising a molecule as defined in anyone of claims 10 to 13, and a pharmaceutically acceptable carrier.
